# EUROPEAN PATENT APPLICATION

(11) **EP 3 875 844 A1**
(43) Date of publication of application: **08.09.2021**
(21) Application number: 18938611.3
(22) Date of filing: 02.11.2018
(51) Int. Cl.: F21S 8/06, F21V 19/00, A61B 90/30

(54) **OPERATING ROOM LIGHTING SYSTEM, CONTROL METHOD AND DEVICE THEREOF, AND STORAGE MEDIUM**

(71) Applicant: Nanjing Mindray Bio-Medical Electronics Co., Ltd., Nanjing, Jiangsu 211111 (CN)
(72) Inventor: CHEN, Xiaokai, Nanjing, Jiangsu 211111 (CN); WEI, Chao, Nanjing, Jiangsu 211111 (CN); WANG, Lei, Nanjing, Jiangsu 211111 (CN); YUAN, Yi, Nanjing, Jiangsu 211111 (CN); WANG, Wenlong, Nanjing, Jiangsu 211111 (CN)
(74) Representative: KIPA AB
(86) International application number: PCT/CN2018/113838
(87) International publication number: WO 2020/087542

(57) **Abstract**

An operating room lighting system (2), a control method and device thereof, and a storage medium. The operating room lighting system (2) comprises: a first operating room light source (21), a second operating room light source (22), and a first sensor (23). The first operating room light source (21) comprises a lamp head (211) and a first controller (212), and the first controller (212) is connected to the first sensor (23). The second operating room light source (22) comprises a lamp head (221) and a second controller (222). A communication link is provided between the first controller (212) and the second controller (222). The first operating room light source (21) and the second operating room light source (22) are respectively configured to illuminate a first area and a second area during operation. The first sensor (23) is configured to collect occlusion information of the first region. The first controller (212) is configured to send the occlusion information of the first area through the communication link. The second controller (222) is configured to receive occlusion information of the first area through the communication link; and adjust the light intensity or the illumination angle of the lamp head (221) of the second operation room light source (22) according to the occlusion information.

## Description

### Technical Field

This disclosure relates to the field of medical technologies, and in particular to an operation room lighting system, a control method and device thereof, and a storage medium.

### Background

Surgical shadowless lamps (abbreviated as surgical lamps) are necessary devices commonly used in an operation room, which are used to provide illumination for a surgical site of a patient and thus provide a good observation environment for surgeons. The surgical lamp is generally located above or diagonally above the surgical site of the patient, and the surgeon is between the surgical lamp and the surgical site of the patient, and therefore the body of the surgeon, especially the head, is a main factor affecting the lighting of the surgical lamp. The surgical lamp has a large lamp head area and a large light source light-emitting area. The large light-emitting area may be used to reduce or eliminate an umbra region generated by the occlusion of body parts such as the head of the surgeon, and minimize the ghost image as far as possible. Although there is no umbra region under the surgical lamp, the illuminance of the surgical site will decrease by about 30% to 50% due to occlusion of the body of the surgeon, which may have a certain negative effect on the observation of the surgeon.

### Summary of the Invention

In view of this, embodiments of this disclosure provide an operation room lighting system, a control method and device thereof, and a storage medium, which can perform effective illuminance compensation for a region in which light rays are occluded during illumination.

An embodiment of this disclosure provides a operation room lighting system, which comprises: a first operation room light source, a second operation room light source, and a first sensor, wherein the first operation room light source comprises a lamp head and a first controller, the first controller being connected to the first sensor; the second operation room light source comprises a lamp head and a second controller; a communication link is provided between the first controller and the second controller;
the first operation room light source is configured to illuminate a first region during operation;
the second operation room light source is configured to illuminate a second region during operation;
the first sensor is configured to collect occlusion information of the first region;
the first controller is configured to send the occlusion information of the first region through the communication link; and
the second controller is configured to receive the occlusion information of the first region through the communication link;
and adjust the light intensity or the illuminate angle of the lamp head of the second operation room light source according to the occlusion information, such that the light intensity of the overlapping region between the first region and the second region changes, and/or the range of the overlapping region between the first region and the second region changes.

An embodiment of this disclosure further provides a control method for a operation room lighting system, the operation room lighting system comprising: a first operation room light source, a second operation room light source, and a first sensor, wherein the first operation room light source comprises a lamp head and a first controller, the first controller being connected to the first sensor; the second operation room light source comprises a lamp head and a second controller; a communication link is provided between the first controller and the second controller; the method comprising:
collecting, by the first sensor, occlusion information of a first region during the illumination of the first operation room light source and the second operation room light source, wherein the first region is an illumination region corresponding to the first operation room light source, and an illumination region corresponding to the second operation room light source is a second region;
sending, by the first controller, the occlusion information of the first region through the communication link; and
receiving, by the second controller, the occlusion information of the first region through the communication link, and adjusting the light intensity or the illumination angle of the lamp head of the second operation room light source according to the occlusion information, such that the light intensity of the overlapping region between the first region and the second region changes, and/or the range of the overlapping region between the first region and the second region changes.

An embodiment of this disclosure further provides a control device for a operation room lighting system, the control device being applied to the operation room lighting system and comprising:
a memory configured to store a control program of the operation room lighting system; and
a processor configured to execute the program that, when executed, implements a control method for a operation room lighting system provided in the embodiment of this disclosure.

An embodiment of this disclosure further provides a storage medium, comprising a stored program that, when executed, implements a control method for a operation room lighting system provided in the embodiment of this disclosure.

The operation room lighting system provided in the embodiment of this disclosure includes a first operation room light source and a second operation room light source. A first controller in the first operation room light source may transmit occlusion information to a second controller in the second operation room light source through a provided communication link, so that the second controller can perform, based on the occlusion information shared by the first controller, illuminance compensation on a first region illuminated by the first operation room light source. As such, the light intensity of the overlapping region between the first region and a second region illuminated by the second operation room light source changes, and/or the range of the overlapping region between the first region and the second region changes, thereby improving user experience.

### Brief Description of the Drawings

FIG. 1 is a schematic diagram of an alternative structure of a surgical lamp provided in the related art;
FIG. 2 is a schematic diagram of an alternative composition structure of an operation room lighting system provided by an embodiment of this disclosure;
FIG. 3 is a schematic diagram of a composition structure of a first operation room light source provided with an infrared detector;
FIG. 4 is a schematic diagram of controlling a lighting state of a lighting assembly in a lamp head of a first operation room light source provided by an embodiment of this disclosure;
FIG. 5 is a schematic diagram of controlling a lighting state of an operation room lighting system by a terminal provided by an embodiment of this disclosure;
FIG. 6 is a schematic diagram of a composition structure of an operation room lighting system provided by an embodiment of this disclosure;
FIG. 7 is a schematic flowchart of a control method for an operation room lighting system provided by an embodiment of this disclosure;
FIG. 8 is a first schematic diagram of an implementation for determining an occlusion area provided by an embodiment of this disclosure;
FIG. 9 is a second schematic diagram of an implementation for determining an occlusion area provided by an embodiment of this disclosure; and
FIG. 10 is a schematic diagram of a composition structure of a control device for an operation room lighting system provided by an embodiment of this disclosure.

### Detailed Description of Embodiments

This disclosure will be further described below in detail in conjunction with the accompanying drawings and the embodiments. It should be understood that the embodiments provided herein are merely intended to explain this disclosure, and are not intended to limit this disclosure. In addition, the embodiments provided below are used to implement some embodiments of this disclosure, but not all embodiments for implementing this disclosure. In the case of no conflict, the technical solutions recorded in the embodiments of this disclosure may be implemented in any combination.

It should be noted that, in the embodiments of this disclosure, the terms "comprise", "include" or any other variation thereof are intended to cover non-exclusive inclusion, so that a method or device comprising a series of elements comprises not only explicitly recorded elements, but also other elements not explicitly listed, or elements inherent in implementing the method or device. In the absence of more restrictions, the element defined by the phrase "comprising a/an ..." does not exclude the presence of a further related element (for example, steps in the method or units in the system, wherein the unit may be a partial circuit, a partial processor, a partial program, software, or the like) in the method or system that comprises the element.

It should be noted that the term "first/second" in the embodiments of this disclosure is only used to distinguish similar objects, and does not represent specific order for the objects. It may be understood that "first/second" may be interchanged for specific order or chronological order when allowed. It should be understood that the objects distinguished by "first/second" may be interchangeable where appropriate, so that the embodiments of this disclosure described herein can be implemented in an order other than that illustrated or described herein.

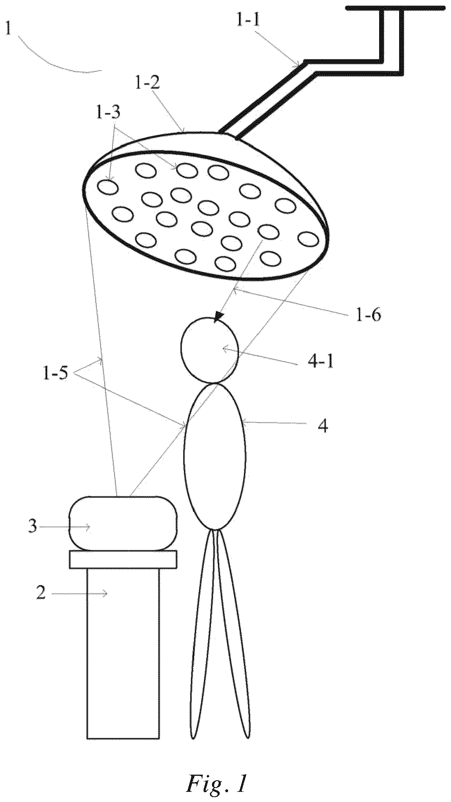

Before describing the operation room lighting system in the embodiments of this disclosure, the operation room lighting system in the related art is described first. In practical application, the operation room lighting system may be a surgical lamp used by surgeons during surgery. FIG. 1 is a schematic diagram of an alternative structure of a surgical lamp provided in the related art. Referring to FIG. 1, in a surgical lighting scene, a surgical lamp 1 is located above a surgical table 2 and a patient 3, and a surgeon 4 is located between the surgical lamp 1 and the patient 3. The surgical lamp 1 includes a hanging assembly 1-1 and a lamp head 1-2, where the hanging assembly 1-1 is generally fixed on the ceiling of an operation room for fixing and hanging the lamp head 1-2. The lamp head 1-2 generally includes one or more lighting assemblies 1-3 (such as small bulbs). When the surgical lamp 1 is working, the lighting assemblies 1-3 emit lighting rays (such as rays 1-5) to illuminate a surgical site of the patient 3. In this case, the surgeon 4 is usually at a location rather close to the surgical site of the patient for more convenient observation and operation. Especially, in order to see clearly the deep cavity in the body of the patient, the eyes of the surgeon 4 overlap with some lighting rays, that is, the head 4-1 or a part of the shoulder of the surgeon is within the illumination region of the lamp head 1-2. In this way, the head 4-1 of the surgeon occludes some lighting rays (such as rays 1-6), which causes the change of illuminance of the surgical site of the patient. In practical application, the illuminance of the surgical site will decrease by about 30% to 50% due to occlusion of the body of the surgeon, which may have a certain negative effect on the observation of the surgeon. In practical application, in order to reduce the effect on the surgery caused by the occlusion of the body of the surgeon, illuminance of the lamp head of the surgical lamp may be increased, but the illuminance of the lamp head cannot be increased indefinitely.

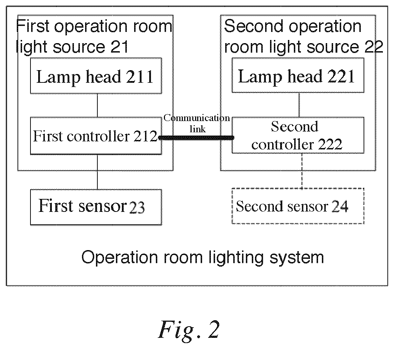

Next, the operation room lighting system provided in the embodiment of this disclosure is described. The operation room lighting system provided in the embodiment of this disclosure may be applied to the lighting during surgery of surgeons in the operation room. FIG. 2 is a schematic diagram of an alternative composition structure of an operation room lighting system provided by an embodiment of this disclosure. Referring to FIG. 2, an operation room lighting system 2 provided in the embodiment of this disclosure includes a first operation room light source 21, a second operation room light source 22, and a first sensor 23.

The first operation room light source 21 includes a lamp head 211 and a first controller 212, and the first controller 212 is connected to the first sensor 23. The second operation room light source 22 includes a lamp head 221 and a second controller 222. A communication link is provided between the first controller 212 and the second controller 222.

The first operation room light source 21 is configured to illuminate a first region during operation.

The second operation room light source 22 is configured to illuminate a second region during operation.

The first sensor 23 is configured to collect occlusion information of the first region.

The first controller 212 is configured to send the occlusion information of the first region through the communication link.

The second controller 222 is configured to receive the occlusion information of the first region through the communication link.

The second controller 222 is also configured to adjust a light intensity or an illumination angle of the lamp head of the second operation room light source according to the occlusion information, such that a light intensity of an overlapping region between the first region and the second region changes, and/or a range of an overlapping region between the first region and the second region changes.

Herein, in practical application, the first sensor 23 connected to the first controller 212 may be arranged outside or inside the first operation room light source 21, and the first sensor is configured to perform occlusion detection on light rays within an illumination range of the lamp head 211.

In the lighting process using the operation room lighting system, the first region illuminated by the first operation room light source 21 may be a surgical region in which the surgeon performs a surgery, that is, a target region, while the second region illuminated by the second operation room light source 22 may be the same as or different from the first region.

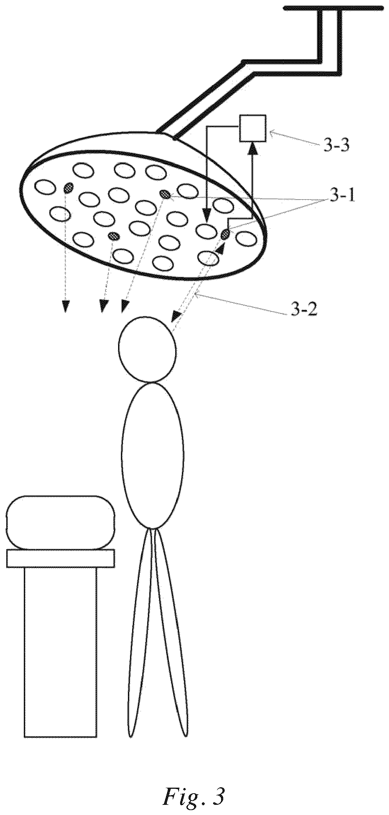

In an embodiment, the first sensor 23 may be an infrared detector, and there are a plurality of infrared detectors. FIG. 3 is a schematic diagram of a composition structure of a first operation room light source provided with an infrared detector. Referring to FIG. 3, a plurality of infrared detectors (as shown by 3-1 in FIG. 3) are provided evenly or non-evenly in the first operation room light source, and a controller 3-3 may control a lighting state (light intensity/illumination angle) of a lighting assembly in the lamp head. The controller 3-3 further controls the infrared detector to emit an infrared signal, and determines whether the emitted infrared signal is occluded by detecting the received infrared signal. For example, the controller may determine that occlusion exists by detecting a received infrared signal 3-2 as shown in the figure. Taking a plurality of infrared detectors that are evenly provided in the first operation room light source as an example, an occlusion situation of illumination light rays of the first operation room light source and a corresponding occlusion region may be predicted by acquiring the number and corresponding positions of the infrared detectors whose infrared signals are occluded (that is, occlusion is detected). For example, 10 infrared detectors are evenly provided in the first operation room light source, and the first controller controls the 10 infrared detectors to perform occlusion detection, and finds that three infrared detectors in the central region are occluded. In this case, it may be learned that about 30% of the light rays of the first operation room light source are occluded, and the occlusion region is in the central illumination region.

Based on the above embodiments of this disclosure, in practical application, the occlusion information includes: the number of the first sensor (infrared detectors) that has detected an obstruction.

Correspondingly, when it is determined that the number of the first sensor that has detected an obstruction reaches a preset number threshold, the second controller is further configured to increase the light intensity of the lamp head of the second operation room light source, such that the light intensity of the overlapping region between the first region and the second region is increased; and/or to adjust the illumination angle of the lamp head of the second operation room light source, such that the range of the overlapping region between the first region and the second region is increased.

Herein, during actual implementation, the number of the first sensor may be set based on actual needs. When the number of the first sensor that has detected an obstruction reaches the preset number threshold, it is determined that illuminance compensation is needed to be performed on the first region, and the light intensity of the lamp head of the second operation room light source is increased, such that the light intensity of the overlapping region between the first region and the second region is increased. For example, when the number of the first sensor that has detected an obstruction reaches the preset number threshold, a coefficient of the light intensity of the lamp head of the second operation room light source is adjusted, for example, increased by 30%. Alternatively, a corresponding adjustment ratio is set according to how much the number of the first sensor that has detected an obstruction exceeds the preset number threshold. For example, when the number of the first sensor is 20, the preset number threshold is 5, and the number of the first sensor that has detected an obstruction is 5, the light intensity of the lamp head of the second operation room light source is increased to 120% of a standard light intensity (a value during initialization of the lamp head). When the number of the first sensor that has detected an obstruction is 10, that is, larger than the preset number threshold by 5, the light intensity of the lamp head of the second operation room light source is increased to 150% of the standard light intensity.

In an embodiment, when the number of the first sensor that has detected an obstruction reaches the preset number threshold, the second controller may further adjust the illumination angle of the lamp head of the second operation room light source, such that the range of the overlapping region between the first region and the second region is increased. That is, the second region illuminated by the second operation room light source overlaps with the first region as much as possible, thereby performing the illuminance compensation on the first region.

During actual implementation, the second controller may dynamically adjust the lighting state (light intensity and illumination angle) of the second operation room light source according to the change of the occlusion information of the first region sent by the first controller. Therefore, when the occlusion information of the first region sent by the first controller again indicates that an occlusion degree is reduced (for example, the number of the first sensor that has detected an obstruction is reduced) to be less than a preset threshold, the second controller decreases the light intensity of the lamp head of the second operation room light source, such that the light intensity of the overlapping region between the first region and the second region is decreased. For example, when the occlusion information received by the second controller again indicates that there is no occlusion in the first region, the lamp head of the second operation room light source is controlled to be turned off. In one embodiment, when the occlusion information of the first region sent by the first controller again indicates that an occlusion degree is reduced to be less than a preset threshold, the illumination angle of the lamp head of the second operation room light source may also be adjusted, such that the range of the overlapping region between the first region and the second region is decreased. For example, when the occlusion information received by the second controller again indicates that there is no occlusion in the first region, the illumination angle of the lamp head of the second operation room light source is adjusted, such that no overlapping region exists between the first region and the second region.

In an embodiment, the occlusion information of the first region includes: an occlusion area corresponding to the first region. Correspondingly, when it is determined that the size of the occlusion area reaches a preset area threshold, the second controller is further configured to increase the light intensity of the lamp head of the second operation room light source, such that the light intensity of the overlapping region between the first region and the second region is increased; and/or to adjust the illumination angle of the lamp head of the second operation room light source, such that the range of the overlapping region between the first region and the second region is increased.

The occlusion area herein may be detected using a three-dimensional time-of-flight image sensor based on time-of-flight detection, or a three-dimensional structured light vision sensor based on structured light detection, or a binocular stereo vision sensor based on binocular stereo vision detection.

Specifically, the second controller may determine to increase the coefficient of the light intensity of the lamp head of the second operation room light source according to a ratio of the occlusion area to the area of the illumination region of the first operation room light source. For example, when the ratio of the occlusion area to the area of the illumination region of the first operation room light source is 1/2, the coefficient of the light intensity of the lamp head of the second operation room light source is increased to 0.5, that is, the light intensity of the lamp head of the second operation room light source is adjusted to 150% of the standard light intensity.

During actual implementation, when the occlusion information of the first region sent by the first controller again indicates that the occlusion degree is reduced (for example, the occlusion area is decreased) to be less than the preset threshold, the second controller decreases the light intensity of the lamp head of the second operation room light source, such that the light intensity of the overlapping region between the first region and the second region is decreased; and/or the second controller adjusts the illumination angle of the lamp head of the second operation room light source, such that the range of the overlapping region between the first region and the second region is decreased.

In an embodiment, the lamp head of the first operation room light source includes a plurality of lighting assemblies. When the first controller determines that occlusion exists in the first region illuminated by the first operation room light source based on the occlusion detection by the first sensor, the first controller is further configured to adjust the light intensities and/or the illumination angles of the plurality of lighting assemblies according to the occlusion information, thereby performing the illuminance compensation on the first region.

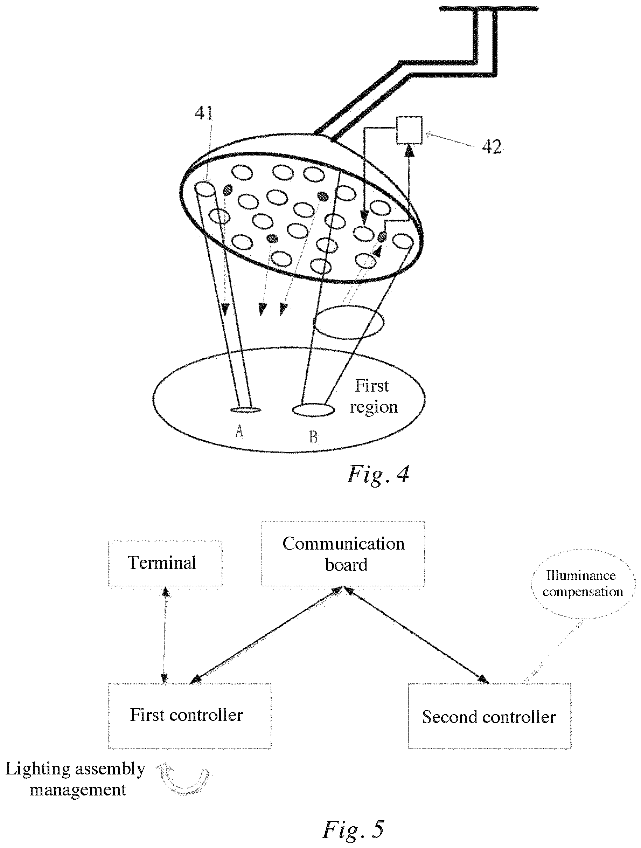

For example, referring to FIG. 4, FIG. 4 is a schematic diagram of controlling a lighting state of a lighting assembly in a lamp head of a first operation room light source provided by an embodiment of this disclosure. In FIG. 4, when the first controller 42 determines, by means of the first sensor, that illumination light rays to a region B of the first region are occluded, the first controller may increase the light intensity of the lighting assembly (such as 41) of the first operation room light source, or adjust the illumination angle of the lighting assembly with an illumination region outside the region B, such that there is an overlap between the illumination region and the region B. For example, the illumination angle of a lighting assembly 41 is adjusted, such that there is an overlap between the illumination region A and the illumination region B.

In an embodiment, the surgeon or other medical care personnel may establish a communication connection to the first controller and/or the second controller by means of a mobile terminal, and send control instructions to the first controller and/or the second controller to control the lighting state of the lamp head of the first operation room light source and/or the second operation room light source. FIG. 5 is a schematic diagram of controlling a lighting state of an operation room lighting system by a terminal provided by an embodiment of this disclosure. Referring to FIG. 5, the terminal may send a control instruction to the first controller through a wireless communication connection to the first controller. The first controller adjusts the lighting states of a plurality of lighting assemblies thereof (for example, increases the light intensity of each of the lighting assemblies) based on the received control instruction, and the second controller cooperates with the first operation room light source to perform the illuminance compensation based on the occlusion information shared by the first controller.

In an embodiment, the operation room lighting system further includes a second sensor 24 connected to the second controller 222.

The second sensor is configured to collect occlusion information of a second region.

The second controller is further configured to send the occlusion information of the second region through the communication link.

The first controller is further configured to receive the occlusion information of the second region through the communication link.

The first controller also adjust the light intensity or the illumination angle of the lamp head of the first operation room light source according to the occlusion information of the second region and the occlusion information of the first region, such that the light intensity of the overlapping region between the first region and the second region changes, and/or the range of the overlapping region between the first region and the second region changes.

Herein, in practical application, after receiving the occlusion information of the second region, the first controller may adjust the light intensity or the illumination angle of the lamp head of the first operation room light source by using the following method.

The first controller compares the occlusion information of the second region with the occlusion information of the first region, and adjust the light intensity or the illumination angle of the lamp head of the first operation room light source based on a comparison result. For example, the occlusion information is the occlusion area. If the occlusion area of the second region is greater than that of the first region, the illuminance compensation is preferably performed on the first region, and the light intensity of the lamp head of the first operation room light source is increased, such that the light intensity of the first region is increased. If the occlusion area of the second region is less than that of the first region, the illuminance compensation is preferably performed on the second region, and the illumination angle of the lamp head of the first operation room light source is adjusted, such that the area of the overlap region between the first region and the second region is increased.

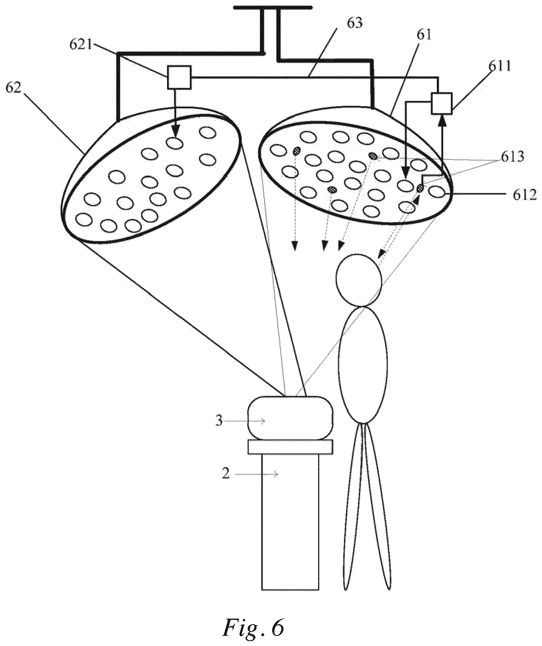

Next, a control method for an operation room lighting system provided in the embodiments of this disclosure is described based on the operation room lighting system provided in the embodiment of this disclosure. FIG. 6 is a schematic diagram of a composition structure of an operation room lighting system provided in an embodiment of this disclosure. Referring to FIG. 6, the operation room lighting system provided in the embodiment of this disclosure includes a primary lamp head 61 (corresponding to the first operation room light source in the foregoing embodiment of this disclosure) and a secondary lamp head 62 (corresponding to the second operation room light source in the foregoing embodiment of this disclosure). The primary lamp head 61 is provided with a primary controller 611, the secondary lamp head 62 is provided with a secondary controller 621, and the primary controller 611 and the secondary controller 621 may communicate through a communication link 63. The primary lamp head 61 is further provided with a plurality of lighting assemblies (small bulbs) 612, and a plurality of occlusion sensors (infrared detectors) 613 evenly provided around the lighting assemblies. The occlusion sensor 613 is connected to the primary controller 611 to receive an occlusion detection instruction of the primary controller 611 to perform light ray occlusion detection on the illumination region of the primary lamp head 61 (that is, the first region) and return occlusion information to the primary controller 611.

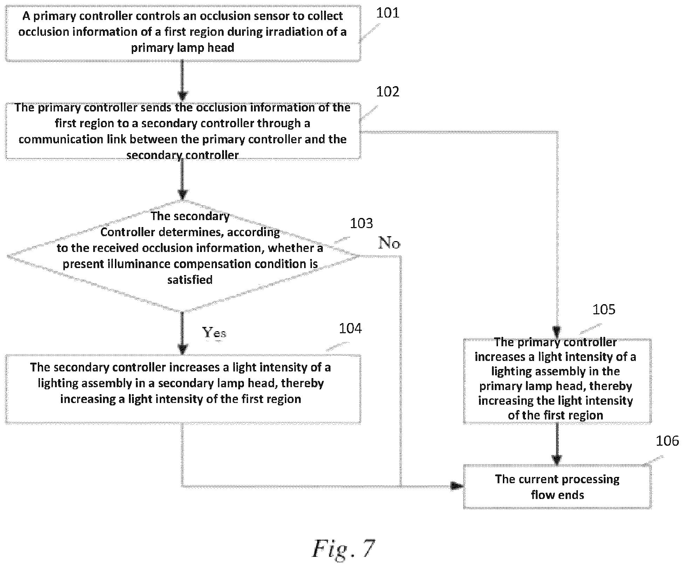

FIG. 7 is a schematic flowchart of a control method for an operation room lighting system provided by an embodiment of this disclosure. With reference to FIGs. 6 and 7, the control method for an operation room lighting system provided in the embodiment of this disclosure is described, which includes the following steps.

At step 101: the primary controller controls the occlusion sensor to collect the occlusion information of the first region during illumination of a primary lamp head.

Herein, the occlusion information of the first region includes the number of the occlusion sensor that has detected an obstruction, and it may be determined, through the number, whether light rays for illuminating the first region are occluded, and how is a corresponding occlusion situation when there is the occlusion. For example, if 10 occlusion sensors are evenly provided in the primary lamp head, and it is found that three of the occlusion sensors have detected light ray occlusion, it may be learned that the light rays for illuminating the first region are occluded, and the corresponding occlusion situation is that about 1/3 of the illumination region is occluded. In practical application, the first region is a surgery region in which the surgeons perform surgery. Before the surgery, when the operation room lighting system is initialized, the illumination regions of the primary lamp head and the secondary lamp head are both the first region.

At step 102: the primary controller sends the occlusion information of the first region to the secondary controller through the communication link between the primary controller and the secondary controller, and performs step 105.

Herein, the communication connection between the primary controller and the secondary controller may be a wired connection or a wireless connection.

At step 103: the secondary controller determines, according to the received occlusion information, whether a preset illuminance compensation condition is satisfied, and when the condition is satisfied, performs step 104; and when the conditions is not satisfied, performs step 106.

Herein, the secondary controller may determine, in the following manner, whether the preset illuminance compensation condition is satisfied.

The secondary controller determines whether the number of the occlusion sensor that has detected an obstruction in the primary lamp head reaches a preset number threshold, determines that the preset illuminance compensation condition is satisfied when the number reaches the preset number threshold; and determines that the preset illuminance compensation condition is not satisfied when the number does not reach the preset number threshold.

Step 104: the secondary controller increases a light intensity of a lighting assembly in the secondary lamp head, thereby increasing a light intensity of the first region.

Herein, in practical application, the secondary controller determines the illuminance compensation situation based on the occlusion information of the first region shared by the primary controller, and actively performs the illuminance compensation on the first region, thereby implementing the coordination of the illuminance compensation with the primary lamp head during lighting of the first region, and improving user experience.

During actual implementation, an adjustment increment of the light intensity of the secondary lamp head performed by the secondary controller may be preset based on an actual situation, for example, 20%.

At step 105: the primary controller increases a light intensity of a lighting assembly in the primary lamp head, thereby increasing the light intensity of the first region.

Herein, in practical application, regardless of whether the occlusion information of the first region satisfies the preset illuminance compensation condition, the primary controller controls the primary lamp head to perform the illuminance compensation on the first region.

At step 106: the current processing flow ends.

The operation room lighting system provided in the embodiment of this disclosure continues to be described. In practical application, considering characteristics that the position of an obstruction such as a surgeon, especially the head of the surgeon, constantly changes during surgery, and those obstructions with different relative positions, shapes, and areas occlude different light paths of the surgical lamp, a sensor is used to detect the obstruction between the surgical lamp and the surgical site in real time. After the relative position, the shape and the size of the obstruction and the relative position information of the surgical site are measured, spatial distribution of the light rays outputted by the surgical lamp may be determined based on information such as the relative position, the shape, the size, and the light intensity distribution of the surgical lamp. Then, an occlusion area ratio may be determined according to the above characteristic information and the light intensity distribution information, that is, an occlusion degree of the obstruction to the light path of the at least one lighting assembly is determined, and an illuminance change on the surgical site caused by the occlusion is further determined. In addition, a light intensity compensation strategy of the lighting system can be determined according to the occlusion degree of the obstruction to the light path of the at least one lighting assembly. Finally, it can be ensured that the illuminance of the surgical site of a patient basically remains constant no matter how the obstruction under the surgical lamp changes, so that the surgical lamp has an optimal shadowless effect.

In an embodiment, a data collection frequency of the sensor is greater than a first frequency threshold. When the data collection frequency of the sensor is high enough, and a data processing speed of the controller and a frequency of light intensity compensation for the lighting assembly are high enough, the illuminance of the surgical site can be compensated for nearly in real time according to the occlusion degree of the obstruction. In addition, the controller may accurately evaluate the occlusion degree of the obstruction to the light path of the lighting assembly according to the above characteristic information and the light intensity distribution information, and then may accurately compensate for the light intensity of the lighting assembly in real time according to the occlusion degree of the obstruction to the light path of the lighting assembly. As such, regardless of whether the obstructions such as the head or the body of the surgeon enter or leave an illumination region of the surgical lamp, or are far away from or close to the surgical site, the surgical site can be fully illuminated, thereby improving the shadowless lighting effect of the surgical lamp.

In an example, a diameter of the lamp head of the surgical lamp is about 500 mm to 700 mm, and the light rays are emitted from the lamp head and converges at a position about 1000 mm away from the lamp head, to form a light spot with a diameter of about 200 mm. In order to achieve a good shadowless effect of the light emitted by the surgical lamp, light distribution at a light emission position of the lamp head of the surgical lamp is relatively even. In this way, from the lamp head of the surgical lamp to the surgical site, the spatial distribution of the light rays is in the shape of a conical light beam, or a light beam with a large cross-sectional area at a position close to the lamp head and a small cross-sectional area at a position far from the lamp head and close to the surgical site. As a result, the obstructions such as the head of the surgeon occlude the light rays in different spatial positions. In order to accurately evaluate how many light lays the obstructions occlude, it is necessary to accurately determine the spatial positions of the obstructions in the space of the beams of the surgical lamp and the occlusion area ratio.

In a first implementation in which the controller determines the occlusion area ratio, the controller may be configured to determine a first cross section and a second cross section based on the above characteristic information and the light intensity distribution information, and determine the occlusion area ratio based on the first cross section and the second cross section. The first cross section is the maximum occlusion cross section of the obstruction relative to the light path of the lighting system, the second cross section is a cross section obtained through intersection of a plane of the first cross section and the light path of the at least one lighting assembly, and the occlusion area ratio is a ratio of an area of the first cross section to an area of the second cross section.

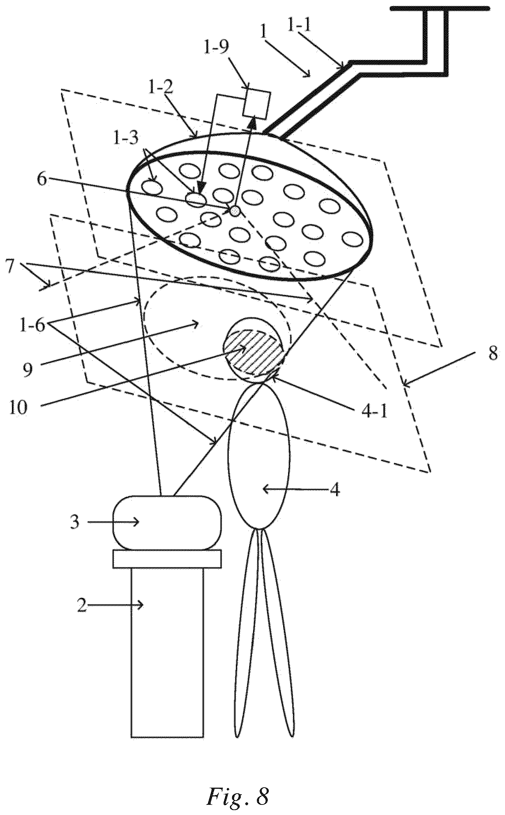

The first implementation of determining the occlusion area ratio by a controller 1-9 is described below with reference to FIG. 8. FIG. 8 is a schematic diagram of an implementation of determining an occlusion area ratio for a structure of an operation room lighting system of an embodiment of this disclosure. As shown in FIG. 8, a sensor 6 is located in a lamp head system 1-2, and the sensor 6 has a certain detection range 7. The detection range 7 may cover most of a region under the lamp head of the surgical lamp, and information such as a position, a shape, and a size of a head 4-1 of a surgeon under the lamp head may be detected. Light rays emitted from the lamp head of the surgical lamp forms a conical light path 1-6 in the space under the lamp head, and the controller 1-9 may store light intensity distribution information of the light beam 1-6 therein. When obstructions such as the head 4-1 of the surgeon are in the space under the lamp head of the surgical lamp, the sensor 6 may detect spatial position information, shape information, and size information of the obstructions relative to the light path 1-6. The controller 1-9 may comprehensively determine a cross section of the obstruction with the greatest occlusion effect relative to the light path of the at least one lighting assembly according to information from the sensor 6 and the light intensity distribution information of the light path 1-6, that is, determine the first cross section. The controller may further determine the second cross section according to a position of the first cross section and the light intensity distribution information of the light path 1-6. For example, referring to FIG. 8, a first cross section 10 is located on a plane 8, and the plane 8 is parallel to an emitting plane of the lamp head of the surgical lamp. A second cross section 9 is a cross section of the light path 1-6 on the plane 8. The first cross section 10 and the second cross section 9 are both elliptical, and the first cross section 10 is completely in the second cross section 9. In this case, an occlusion area ratio may be obtained, which is a ratio of an area of the first cross section 10 to an area of the second cross section 9. Herein, in order to keep illuminance of the surgical site basically constant, the controller 1-9 may control, according to the occlusion area ratio, a light intensity of the at least one lighting assembly 1-3 to increase, or an illumination angle of the at least one lighting assembly 1-3 to change.

In a second implementation of determining the occlusion area ratio by the controller 1-9, the controller 1-9 may be configured to determine an equivalent occlusion region of the obstruction on an emission plane of the light path of the lighting system 11 according to the above characteristic information and the light intensity distribution information, and determine the occlusion area ratio according to the equivalent occlusion region. The equivalent occlusion region is a projection region of the obstruction on the emission plane of the light path of the lighting system 11, and the occlusion area ratio is a ratio of an area of the equivalent occlusion region to an area of the emission plane of the light path of the lighting system 11.

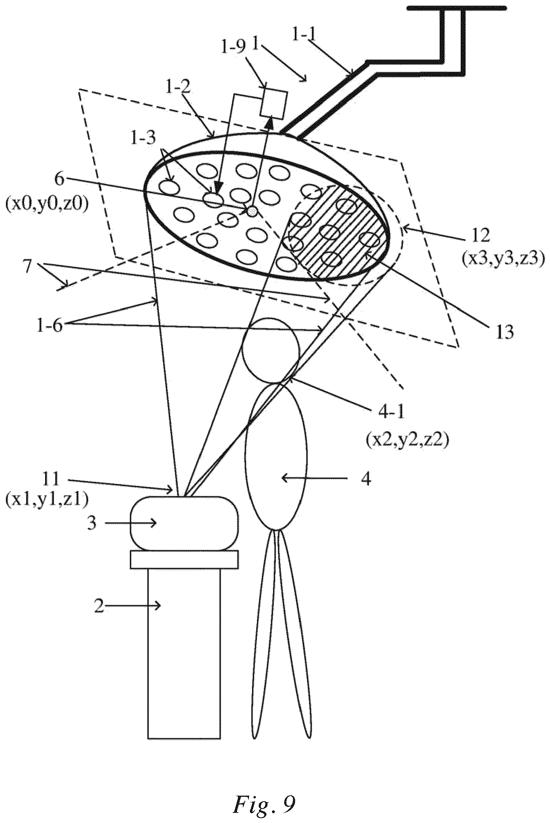

The second implementation of determining the occlusion area ratio by the controller 1-9 is described below with reference to FIG. 9. FIG. 9 is a schematic diagram of a second implementation of determining an occlusion area ratio for a structure of an operation room lighting system of an embodiment of this disclosure. As shown in FIG. 9, a sensor 6 is located in a lamp head system 1-2, and the sensor 6 has a certain detection range 7. The detection range 7 may cover most of a region under the lamp head of the surgical lamp, information such as a position, a shape, and a size of a head 4-1 of a surgeon under the lamp head may be detected, and position information of a surgical site 11 may also be detected. According to the design characteristics of the surgical lamp, the surgical site 11 is generally located at the convergence center of beams of the surgical lamp. In the controller 1-9, the emission plane of the light path of the lighting assembly 1-3 may be set as a reference plane, and the sensor 6 or the center position of the lamp head is a reference coordinate origin (x0, y0, z0). The sensor 6 may first determine coordinates (x1, y1, z1) of the surgical site 11 through detection, then may determine position coordinates (x2, y2, z2) of the center or approximate the center of the obstruction and size information of the obstruction, and then may calculate a position and an area of a projection 12 of the obstruction on the emission plane of the light path of the lighting assembly 1-3 according to a projection relationship. Herein, center coordinates of the projection 12 are (x3, y3, z3). According to the position of the projection 12 on the emission plane of the light path of the lighting assembly 1-3, an equivalent occlusion region of the obstruction on the emission plane of the light path of the at least one lighting assembly 1-3 is calculated. It should be noted that, according to the geometric projection relationship, the projection 12 of the obstruction on the emission plane of the light path of the lighting assembly 1-3 derived from coordinates (x1, y1, z1) is not necessarily completely located within the emission plane of the light path of the lighting assembly 1-3, or may be partially located outside the emission plane of the light path of the lighting assembly 1-3. The equivalent occlusion region herein is a region of the projection 12 within the emission plane of the light path of the lighting assembly 1-3. With reference to FIG. 9, the above equivalent occlusion region is an oblique line region 13. In this case, the occlusion area ratio may be obtained, which is a ratio of an area of the oblique line region 13 to an area of the emission plane of the light path of the lighting assembly 1-3. Herein, in order to keep illuminance of the surgical site basically constant, the controller 1-9 may control, according to the occlusion area ratio, a light intensity of the at least one lighting assembly 1-3 to increase, or an illumination angle of the at least one lighting assembly 1-3 to change.

It should be noted that when the controller 1-9 determines, according to the shape information of the obstruction, that the obstruction is a regular and symmetric object, the coordinate of the center of the obstruction may be determined according to the spatial position information, the size information, and the shape information of the obstruction. When the controller 1-9 determines, according to the shape information of the obstruction, that the obstruction is an irregular object or an asymmetric object, approximate processing may be performed on the shape of the obstruction to obtain a regular and symmetric object shape. Then, the coordinate of the center of the obstruction may be determined according to the spatial position information and the size information of the obstruction. The embodiment of this disclosure does not limit the approximate processing method for the shape of the obstruction.

During actual implementation, the controller 1-9 determines an occlusion area ratio, and may determine a light intensity compensation strategy of the lighting system according to the occlusion area ratio. In an example, the above light intensity distribution information further includes the current light intensity of the at least one lighting assembly 1-3. Light intensity adjustment of the at least one lighting assembly 1-3 includes obtaining a light intensity of the at least one lighting assembly 1-3 obtained after compensation. The controller may be configured to determine the light intensity of the at least one lighting assembly obtained after the compensation according to the occlusion area ratio and the current light intensity of the at least one lighting assembly 1-3. Specifically, the light intensity of at least one lighting assembly 1-3 obtained after the compensation may be obtained by dividing the current light intensity of at least one lighting assembly 1-3 by the occlusion area ratio, and then the at least one lighting assembly 1-3 is controlled to emit light according to the light intensity obtained after the compensation. In this way, it may be basically ensured that the illuminance of the surgical site when the light path of the lighting assembly 1-3 is occluded is consistent with that of the surgical site when the light path of the lighting assembly 1-3 is not occluded.

It should be noted that when the number of the lighting assemblies is greater than 1, the controller may divide the current light intensity of each of the lighting assemblies by the occlusion area ratio to obtain the light intensity of each of the lighting assemblies obtained after compensation, and then control each of the lighting assemblies to emit light according to the light intensity obtained after the compensation.

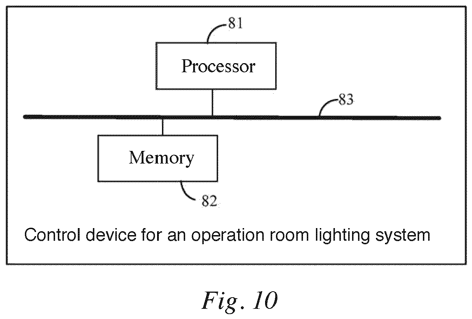

In an embodiment, an embodiment of this disclosure further provides a control device for an operation room lighting system. The control device is applied to the operation room lighting system. Referring to FIG. 10, FIG. 10 is a schematic diagram of an alternative composition structure of a control device for an operation room lighting system provided in an embodiment of this disclosure. It may be understood that the control device for an operation room lighting system in FIG. 10 is only an exemplary structure rather than the entire structure, and a part or all of the structure shown in FIG. 10 may be implemented as required.

The control device for the operation room lighting system provided in the embodiment of this disclosure includes: at least one processor 81 and a memory 82. Various assemblies in the control device for the operation room lighting system are coupled together via a bus system 83. It may be understood that the bus system 83 is configured to implement connection and communication between these assemblies. In addition to a data bus, the bus system 83 further includes a power supply bus, a control bus, and a status signal bus.

It may be understood that the memory 82 may be a volatile memory or a non-volatile memory, or may include both a volatile memory and a non-volatile memory.

The memory 82 in the embodiment of this disclosure is configured to store various types of data to support operation of the control device for an operation room lighting system.

The control method for an operation room lighting system disclosed in the embodiment of this disclosure may be applied to the processor 81 or implemented by the processor 81. The processor 81 may be an integrated circuit chip and has a signal processing capability. During implementation, each step of the software login method for an in-vitro diagnostic apparatus may be completed by means of an integrated logic circuit of hardware in the processor 81 or an instruction in the form of software. The above processor 81 may be a general-purpose processor, a digital signal processor (DSP), or other programmable logic devices, a discrete gate or a transistor logic device, a discrete hardware component, or the like. The processor 81 may implement or execute various methods, steps, and logic block diagrams disclosed in the embodiments of this disclosure. The general-purpose processor may be a microprocessor, any conventional processor, or the like. The steps of the method disclosed in conjunction with the embodiments of this disclosure may be directly performed by a hardware decoding processor, or performed by a combination of hardware and software modules in the decoding processor.

In an embodiment, the memory is configured to store a control program of the operation room lighting system.

The processor is configured to execute the program that, when executed, implements a control method for a operation room lighting system provided in the embodiment of this disclosure.

Correspondingly, an embodiment of this disclosure further provides a readable storage medium, which comprises: a mobile storage device, a random access memory (RAM), a read-only memory (ROM), a magnetic disk or an optical disk and other media that can store program code. The readable storage medium stores an executable instruction.

The executable instruction, when executed by the processor, is used to implement the control method for a operation room lighting system.

All or some of the steps of the embodiments may be completed by a program that instructs related hardware. The program may be stored in a computer readable storage medium. When the program is executed, the steps comprising the above method embodiments are performed. The foregoing storage medium comprises: a mobile storage device, a random access memory, a read-only memory, a magnetic disk or an optical disk and other media that can store program code.

Alternatively, if implemented in the form of a software function module and sold or used as an independent product, the above integrated unit of this disclosure may also be stored in a computer readable storage medium. Based on such an understanding, the technical solutions in the embodiments of this disclosure essentially, or the part contributing to the related art may be implemented in a form of a software product. The computer software product is stored in a storage medium, comprising several instructions for instructing a computer device (which may be a personal computer, a server, or a network device) to perform all or some of the methods in the embodiments of this disclosure. The foregoing storage medium comprises: a mobile storage device, a RAM, a ROM, a magnetic disk or an optical disk and other media that can store program code.

The above descriptions are merely specific embodiments of this disclosure, but the scope of protection of this disclosure is not limited thereto. Changes or substitutions readily figured out by those skilled in the art within the technical scope disclosed in this disclosure shall fall within the scope of protection of this disclosure. Therefore, the scope of protection of this disclosure shall be subject to the scope of protection of the claims.

## Claims

1. An operation room lighting system, **characterized in that** comprising: a first operation room light source, a second operation room light source, and a first sensor, wherein the first operation room light source comprises a lamp head and a first controller, the first controller being connected to the first sensor; the second operation room light source comprises a lamp head and a second controller; a communication link is provided between the first controller and the second controller;
the first operation room light source is configured to illuminate a first region during operation;
the second operation room light source is configured to illuminate a second region during operation;
the first sensor is configured to collect occlusion information of the first region;
the first controller is configured to send the occlusion information of the first region through the communication link; and
the second controller is configured to receive the occlusion information of the first region through the communication link;
and adjust a light intensity or an illumination angle of the lamp head of the second operation room light source according to the occlusion information, such that a light intensity of an overlapping region between the first region and the second region changes, and/or a range of an overlapping region between the first region and the second region changes.

2. The operation room lighting system of claim 1, **characterized in that** further comprising a second sensor connected to the second controller,
the second sensor is configured to collect occlusion information of the second region;
the second controller is further configured to send the occlusion information of the second region through the communication link; and
the first controller is further configured to receive the occlusion information of the second region through the communication link;
and adjust a light intensity or an illumination angle of the lamp head of the first operation room light source according to the occlusion information of the second region and the occlusion information of the first region, such that the light intensity of the overlapping region between the first region and the second region changes, and/or the range of the overlapping region between the first region and the second region changes.

3. The operation room lighting system of claim 1, **characterized in that**
the occlusion information comprises: an occlusion area corresponding to the first region; and
when it is determined that a size of the occlusion area reaches a preset area threshold, the second controller is further configured to increase the light intensity of the lamp head of the second operation room light source, such that the light intensity of the overlapping region between the first region and the second region is increased;
and/or, to adjust the illumination angle of the lamp head of the second surgical room light source, such that the range of the overlapping region between the first region and the second region is increased.

4. The operation room lighting system of claim 3, **characterized in that**,
when it is determined that the occlusion area decreases to be less than the preset area threshold, the second controller is further configured to decrease the light intensity of the lamp head of the second surgical room light source, such that the light intensity of the overlapping region between the first region and the second region is decreased;
and/or, to adjust the illumination angle of the lamp head of the second operation room light source, such that the range of the overlapping region between the first region and the second region is decreased.

5. The operation room lighting system of claim 3, **characterized in that**,
the first sensor is a three-dimensional time-of-flight image sensor based on time-of-flight detection, or a three-dimensional structured light vision sensor based on structured light detection, or a binocular stereo vision sensor based on binocular stereo vision detection.

6. The operation room lighting system of claim 1, **characterized in that** there are a plurality of the first sensors, and the occlusion information of the first region comprises: the number of the first sensor that has detected an obstruction; and
when it is determined that the number of the first sensor that has detected an obstruction reaches a preset number threshold, the second controller is further configured to increase the light intensity of the lamp head of the second operation room light source, such that the light intensity of the overlapping region between the first region and the second region is increased;
and/or, to adjust the illumination angle of the lamp head of the second operation room light source, such that the range of the overlapping region between the first region and the second region is increased.

7. The operation room lighting system of claim 6, **characterized in that**,
when it is determined that the number of the first sensor that has detected an obstruction decreases to be less than the preset number threshold, the second controller is further configured to decrease the light intensity of the lamp head of the second operation room light source, such that the light intensity of the overlapping region between the first region and the second region is decreased;
and/or, to adjust the illumination angle of the lamp head of the second operation room light source, such that the range of the overlapping region between the first region and the second region is decreased.

8. The operation room lighting system of claim 6, wherein
the occlusion sensor (the first sensor and/or the second sensor) is an infrared detector based on infrared signal detection.

9. The operation room lighting system of claim 1, **characterized in that** the lamp head of the first operation room light source comprises a plurality of lighting assemblies; and
the first controller is further configured to adjust light intensities and/or illumination angles of the plurality of lighting assemblies according to the occlusion information.

10. The operation room lighting system of claim 2, **characterized in that**
the first controller is further configured to compare the occlusion information of the second region with the occlusion information of the first region, and adjust the light intensity or the illumination angle of the lamp head of the first operation room light source based on a comparison result.

11. A control method for an operation room lighting system, the operation room lighting system comprising: a first operation room light source, a second operation room light source, and a first sensor, wherein the first operation room light source comprises a lamp head and a first controller, the first controller being connected to the first sensor; the second operation room light source comprises a lamp head and a second controller; and a communication link is provided between the first controller and the second controller; **characterized in that** the method comprising:
collecting, by the first sensor, occlusion information of a first region during illumination of the first operation room light source and the second operation room light source, wherein the first region is an illumination region corresponding to the first operation room light source, and an illumination region corresponding to the second operation room light source is a second region;
sending, by the first controller, the occlusion information of the first region through the communication link; and
receiving, by the second controller, the occlusion information of the first region through the communication link, and adjusting a light intensity or an illumination angle of the lamp head of the second operation room light source according to the occlusion information, such that a light intensity of an overlapping region between the first region and the second region changes, and/or a range of an overlapping region between the first region and the second region changes.

12. The control method of claim 11, **characterized in that** the operation room lighting system further comprises a second sensor connected to the second controller, and the method further comprises:
collecting, by the second sensor, occlusion information of the second region;
sending, by the second controller, the occlusion information of the second region through the communication link; and
receiving, by the first controller, the occlusion information of the second region through the communication link, and adjusting a light intensity or an illumination angle of the lamp head of the first operation room light source according to the occlusion information of the second region and the occlusion information of the first region, such that the light intensity of the overlapping region between the first region and the second region changes, and/or the range of the overlapping region between the first region and the second region changes.

13. The control method of claim 11, **characterized in that** the occlusion information comprises: an occlusion area corresponding to the first region, and said adjusting, by the second controller, the light intensity or the illumination angle of the lamp head of the second operation room light source according to the occlusion information comprises:
when it is determined that the size of the occlusion area reaches a preset area threshold, increasing, by the second controller, the light intensity of the lamp head of the second operation room light source, such that the light intensity of the overlapping region between the first region and the second region is increased; and/or adjusting, by the second controller, the illumination angle of the lamp head of the second operation room light source, such that the range of the overlapping region between the first region and the second region is increased.

14. The control method of claim 13, **characterized in that** further comprising:
when it is determined that the occlusion area decreases to be less than the preset area threshold, decreasing, by the second controller, the light intensity of the lamp head of the second operation room light source, such that the light intensity of the overlapping region between the first region and the second region is decreased;
and/or adjusting, by the second controller, the illumination angle of the lamp head of the second operation room light source, such that the range of the overlapping region between the first region and the second region is decreased.

15. The control method of claim 13, **characterized in that**
the first sensor is a three-dimensional time-of-flight image sensor based on time-of-flight detection, or a three-dimensional structured light vision sensor based on structured light detection, or a binocular stereo vision sensor based on binocular stereo vision detection.

16. The control method of claim 11, **characterized in that** there are a plurality of the first sensors, and the occlusion information comprises: the number of the first sensor that has detected an obstruction; and said adjusting, by the second controller, the light intensity or the illumination angle of the lamp head of the second operation room light source according to the occlusion information comprises:
when it is determined that the number of the first sensors having detected obstructions reaches a preset number threshold, increasing, by the second controller, the light intensity of the lamp head of the second operation room light source, such that the light intensity of the overlapping region between the first region and the second region is increased;
and/or adjusting, by the second controller, the illumination angle of the lamp head of the second operation room light source, such that the range of the overlapping region between the first region and the second region is increased.

17. The control method of claim 16, **characterized in that**, further comprising:
when it is determined that the number of the first sensors having detected obstructions decreases to be less than the preset number threshold, decreasing, by the second controller, the light intensity of the lamp head of the second operation room light source, such that the light intensity of the overlapping region between the first region and the second region is decreased;
and/or adjusting, by the second controller, the illumination angle of the lamp head of the second operation room light source, such that the range of the overlapping region between the first region and the second region is decreased.

18. The control method of claim 16, **characterized in that**
the occlusion sensor is an infrared detector based on infrared signal detection.

19. The control method of claim 11, **characterized in that** the lamp head of the first operation room light source comprises a plurality of lighting assemblies; and the method further comprises:
adjusting, by the first controller, light intensities and/or illumination angles of the plurality of lighting assemblies according to the occlusion information.

20. The control method of claim 12, **characterized in that** said adjusting, by the first controller, the light intensity or the illumination angle of the lamp head of the first operation room light source according to the occlusion information of the second region and the occlusion information of the first region comprises:
comparing, by the first controller, the occlusion information of the second region with the occlusion information of the first region, and adjusting the light intensity or the illumination angle of the lamp head of the first operation room light source based on a comparison result.

21. A control device for an operation room lighting system, the control device being applied to the operation room lighting system, **characterized in that** comprising:
a memory configured to store a control program of the operation room lighting system; and
a processor configured to execute the program that, when executed, implements a control method for a operation room lighting system of any one of claims 11 to 20.

22. A storage medium, comprising a stored program that, when executed, implements a control method for a operation room lighting system of any one of claims 11 to 20.
